# EUROPEAN PATENT APPLICATION

(11) **EP 3 599 283 A1**
(43) Date of publication of application: **29.01.2020**
(21) Application number: 18000623.1
(22) Date of filing: 25.07.2018
(51) Int. Cl.: C12Q 1/6851, C12Q 1/686, C12Q 1/689

(54) **METHOD FOR ASSESSING FECAL POLLUTION IN WATER**

(71) Applicant: Blue DNA Companion, 63100 Clermont-Ferrand (FR); SUEZ Eau France, 92040 Paris la Défense CEDEX (FR)
(72) Inventor: Dameron, Séverine, 63910 Vertaizon (FR); Chaubron, Franck, 13770 Venelles (FR); Bergeron, Peggy, 64200 Biarritz (FR); Courtois, Sophie, 78230 Le Pecq (FR); Borthaire, Maiena, 64310 Saint-Pée-sur-Nivelle (FR); Fagoaga Otheguy, Pantxika, 64310 Saint-Pée-sur-Nivelle (FR)
(74) Representative: Barbot, Willy

(57) **Abstract**

The present invention relates to oligonucleotides, such as primers and probes, and their use for obtaining amplicon from a living or a viable fecal indicator bacteria-containing sample. The invention also relates to a method for detecting and/or quantifying living and viable fecal indicator bacteria in a water sample.

SEQUENCE LISTING

<110>  BLUE DNA COMPANION
       SUEZ Eau France
       DAMERON, Séverine
       CHAUBRON, Franck
       BERGERON, Peggy
       COURTOIS, Sophie
       BORTHAIRE, Maiena
       FAGOAGA OTHEGUY, Pantxika

<120>  METHOD FOR ASSESSING FECAL POLLUTION IN WATER

<130>  BDC-B-0001-EP1

<160>  32

<170>  PatentIn version 3.5

<210>  1
<211>  89
<212>  DNA
<213>  Escherichia coli

<400>  1
catgtggttt aattcgatgc aacgcgaaga accttacctg gtcttgacat ccacggaagt      60

tttcagagat gagaatgtgc cttcgggaa                                        89


<210>  2
<211>  67
<212>  DNA
<213>  Escherichia coli

<400>  2
cggaagcaac gcgtaaactc gatccgacgc gtccgatcac ctgcgtcaat gtaatgttct      60

gcgacgc                                                                67


<210>  3
<211>  129
<212>  DNA
<213>  Escherichia coli

<400>  3
ggccacggag agttatcagt ttgctgccca gcagctggtt ctgggtatcc ctcaggtggc      60

actgcgtccg cagctgaggg ggattgcgcc ggaggcgtta tgggatgatg gcattccggc     120

ttttctgct                                                             129


<210>  4
<211>  59
<212>  DNA
<213>  Escherichia coli

<400>  4
gcactgtgac cactctgatt gcgctgtgcc tgccggtgag cgtttttgcc accacgctg       59

<210>  5
<211>  188
<212>  DNA
<213>  Enterococcus spp.

<400>  5
gtttccgcct ttcagtgctg cagctaacgc attaagcact ccgcctgggg agtacgaccg      60

caaggttgaa actcaaagga attgacgggg gcccgcacaa gcggtggagc atgtggttta     120

attcgaagca acgcgaagaa ccttaccagg tcttgacatc ctttgaccac tctagagata     180

gagcttcc                                                              188


<210>  6
<211>  93
<212>  DNA
<213>  Enterococcus spp.

<400>  6
gagaaattcc aaacgaactt ggagatagct ggttctctcc gaaatagctt tagggctagc      60

ctcggaattg agaatgatgg aggtagagca ctg                                   93


<210>  7
<211>  23
<212>  DNA
<213>  Artificial Sequence

<220>
<223>  EC1_F

<400>  7
catgtggttt aattcgatgc aac                                              23


<210>  8
<211>  19
<212>  DNA
<213>  Artificial Sequence

<220>
<223>  EC1_R

<400>  8
ttcccgaagg cacattctc                                                   19


<210>  9
<211>  20
<212>  DNA
<213>  Artificial Sequence

<220>
<223>  EC2_F

<400>  9
cggaagcaac gcgtaaactc                                                  20


<210>  10

<211>  22
<212>  DNA
<213>  Artificial Sequence

<220>
<223>  EC2_R

<400>  10
gcgtcgcaga acattacatt ga                                                22


<210>  11
<211>  22
<212>  DNA
<213>  Artificial Sequence

<220>
<223>  EC3_F

<400>  11
ggccacggag agttatcagt tt                                                22


<210>  12
<211>  19
<212>  DNA
<213>  Artificial Sequence

<220>
<223>  EC3_R

<400>  12
agcagaaaag ccggaatgc                                                    19


<210>  13
<211>  18
<212>  DNA
<213>  Artificial Sequence

<220>
<223>  EC4_F

<400>  13
gcatcgtgac caccttga                                                     18


<210>  14
<211>  16
<212>  DNA
<213>  Artificial Sequence

<220>
<223>  EC4_R

<400>  14
cagcgtggtg gcaaaa                                                       16


<210>  15
<211>  14
<212>  DNA
<213>  Artificial Sequence

<220>
<223>  Es1_F

<400>  15
gtttccgccc ttca                                                          14


<210>  16
<211>  22
<212>  DNA
<213>  Artificial Sequence

<220>
<223>  Es1_R

<400>  16
ggaagctcta tctctagagt gg                                                 22


<210>  17
<211>  21
<212>  DNA
<213>  Artificial Sequence

<220>
<223>  Es2_F

<400>  17
gagaaattcc aaacgaactt g                                                  21


<210>  18
<211>  21
<212>  DNA
<213>  Artificial Sequence

<220>
<223>  Es2_R

<400>  18
cagtgctcta cctccatcat t                                                  21


<210>  19
<211>  17
<212>  DNA
<213>  Artificial Sequence

<220>
<223>  EC_Probe1

<400>  19
catccacgga agttttc                                                       17


<210>  20
<211>  21
<212>  DNA
<213>  Artificial Sequence

<220>
<223>  EC_Probe2

<400>  20
acccgacgcg tccgatcacc t                                                21

<210>  21
<211>  24
<212>  DNA
<213>  Artificial Sequence

<220>
<223>  EC_Probe3

<400>  21
tatccctcag gtggcactgc gtcc                                             24

<210>  22
<211>  23
<212>  DNA
<213>  Artificial Sequence

<220>
<223>  EC_Probe4

<400>  22
tgcattatgt ttgccggtat ccg                                              23

<210>  23
<211>  16
<212>  DNA
<213>  Artificial Sequence

<220>
<223>  Es_Probe1

<400>  23
gacatccttt gaccac                                                      16

<210>  24
<211>  29
<212>  DNA
<213>  Artificial Sequence

<220>
<223>  Es_Probe2

<400>  24
tggttctctc cgaaatagct ttagggcta                                        29

<210>  25
<211>  22
<212>  DNA
<213>  Artificial Sequence

<220>
<223>  SA_F

<400>  25
ggttcaaaag tgaaagacgg tc                                               22

<210>  26
<211>  19
<212>  DNA
<213>  Artificial Sequence

<220>
<223>  SA_R

<400>  26
cctctcaggt cggctatgc                                                    19


<210>  27
<211>  18
<212>  DNA
<213>  Artificial Sequence

<220>
<223>  SA_Probe

<400>  27
ggatccgcgc tgcattag                                                     18


<210>  28
<211>  113
<212>  DNA
<213>  Staphylococcus aureus

<400>  28
ggttcaaaag tgaaagacgg tcttgctgtc acttatagat ggatccgcgc tgcattagct      60

agttggtaag gtaacggctt accaaggcaa cgatgcatag ccgacctgag agg            113


<210>  29
<211>  25
<212>  DNA
<213>  Artificial Sequence

<220>
<223>  IC_F

<400>  29
gcaatcgtat tacctcttat cgcag                                             25


<210>  30
<211>  24
<212>  DNA
<213>  Artificial Sequence

<220>
<223>  IC_R

<400>  30
caaccatcgt catcgtcagg aaac                                              24


<210>  31
<211>  17

<212>  DNA
<213>  Artificial Sequence

<220>
<223>  IC_Probe

<400>  31
ggcaaagtcc catcgtt                                                    17


<210>  32
<211>  74
<212>  DNA
<213>  Artificial Sequence

<220>
<223>  IC_ampl

<400>  32
gcaatcgtat tacctcttat cgcagaaaag gcaaagtccc atcgttaaaa gtttcctgac      60

gatgacgatg gttg                                                       74

## Description

### FIELD OF THE INVENTION

The present invention relates to methods and kit for the detection and/or the quantification of fecal indicator organism in a water sample.

### BACKGROUND OF THE INVENTION

Fecal contamination remains, in costal and inland waters around the world, the primary cause of closure for recreational use. Epidemiological studies have shown that recreational exposure from swimming, bathing, surfing, water skiing, tubing, water play with children or eating shellfish in locations impacted by contaminated storm water or any human/animal feces contamination leads to a significant increase in a variety of illnesses including gastrointestinal (GI) illness such as diarrhea or gastroenteritis, less often respiratory illness, and also rash, earache or infected cut.

Scientific advancements in microbiological and statistical methods have been used to evaluate how fecal indicator bacteria (FIB) levels are associated with health effects of primary contact recreation. A clear relationship was established between GI illness rates and the presence of enterococci in both marine and fresh water, and *Escherichia coli* in fresh water. These two microorganisms are used as water-quality indicators in the European Union (Directive 2006/7/EC) and the United-States (2012 Recreational Water Quality Criteria - RWQC published by U.S. Environmental Protection Agency (EPA)).

Guidelines for managing risk in recreational waters established numeric concentration thresholds, either of which would protect the designated use of primary contact recreation and, therefore, would protect the public from exposure to harmful levels of pathogens. The magnitude of the bacterial indicators are described by both a geometric mean (GM) and a statistical threshold value (STV) that should not be exceeded by more than 10 percent of the samples taken. Hence, according to 2012 RWQC, for an estimated illness rate of 35/1,000, culturable enterococci and *E. coli* should stay below a GM of 35 colony forming unit (CFU) per 100 ml and a STV of 130 CFU per 100 ml and a GM of 126 colony forming unit (CFU) per 100 ml and a STV of 410 CFU per 100 ml respectively. According to Directive 2006/7/EC, bathing waters are classified as 'poor quality water' when microbiological enumerations are higher than 330 CFU per 100 ml for enterococci and 900 CFU per 100 ml for *Escherichia coli* for inland waters and higher than 185 CFU per 100 ml for enterococci and 500 CFU per 100 ml for *Escherichia coli* for coastal and transitional waters.

To assess the concentrations of enterococci and *E. coli,* techniques based on defined substrate cultures, such as the IDEXX methods - Colilert™ (Covert et al. APPLIED AND ENVIRONMENTAL MICROBIOLOGY, Vol. 55(10), p.2443-2447) and Enterolert™(Budnick et al. APPLIED AND ENVIRONMENTAL MICROBIOLOGY, Vol. 62(10), p. 3881-3884), are commonly used to quantify indicator levels because of their relative ease of use, low cost, and epidemiological evidence that links such levels to human illness. Methods for quantification of the number of bacteria in a liquid sample are disclosed in patents US 5,753,456, US 5,620,895 and US 5,518,892. They have become the effective industry standard in most countries. But these techniques have at least four drawbacks: (a) they take at least 18 hours to complete (meaning slow reporting to community about risks, or indeed significant divergences between reported and current risks); (b) they require lab personnel to analyze and report results the following day (so weekend staffing issues often make Friday samples problematic); (c) they can introduce user bias (for example colorimetric systems, by relying on visual comparisons with templates, are subject to systematic or arbitrary error); and (d) they are known to have associated specificity issues.

More recently, the new TECTA™ pathogen detection system uses an optical fluorescence sensor, directly coupled to a bacteria culture test, to estimate cell densities in water samples for E. coli, and total coliforms. Fluorescence is used to measure enzyme activity of target organisms, and is automatically converted to concentrations without requiring subjective visual interpretation - in a fraction of the time required by traditional cell-culture techniques. Positive samples were detected in fewer than 18 h, with typical detection times in the 12 h to 15 h range for 1-10 CFU. Moreover, this detection system does not allow the presence of enterococci to be measured.

To match the reduction of time and requested limits of detection, increasing interest is now being directed towards the possible use of molecular microbial analysis methods with shorter reporting times. Thus, methods that directly measure DNA rather than bacterial growth or metabolic activity have been developed. Quantitative polymerase chain reaction (qPCR) techniques, for *Escherichia coli* or *Enterococcus spp.,* have shown promising results and significant correlation with traditional culture-based methods. US Environmental Protection Agency's (US EPA) Method 1611.1 describes a quantitative polymerase chain reaction (qPCR) procedure for the detection of DNA from enterococci bacteria in ambient water matrices based on the amplification and detection of a specific region of the large subunit ribosomal ribonucleic acid (RNA) gene from these organisms. Despite results can be obtained by this method in 3 - 4 hours, allowing same-day notification of recreational water quality, this method rarely yields information about the viability of cells. Moreover, because molecular methods do not distinguish between data from dead and living cells, they nearly always overestimate the enterococci concentrations in comparison to the substrate culture methods.

There is therefore a need for a method allowing to overcome these shortcomings, and in particular for a method allowing to detect and/or quantify rapidly fecal indicator bacteria while improving the accuracy, specificity and sensitivity of existing methods.

### SUMMARY OF THE INVENTION

The inventors have now identified oligonucleotides suitable for amplifying a target nucleic acid sequence allowing to avoid the false-positive results observed in prior art methods. Said oligonucleotides are selected from the group consisting of SEQ ID NO: 1 to 6. The oligonucleotides of the invention can furthermore be used as primers or probes for the detection and/or quantification of fecal indicator bacteria in a sample, notably a water sample. The oligonucleotides of the invention can furthermore be used for discriminating between *Enterococcus spp.* and *Escherichia coli.*

Consequently, the present invention relates to primer pairs and probes suitable for the detection and/or quantification of living and viable *Enterococcus spp.* and *Escherichia coli.* Primer pairs and probes comprising nucleotide sequence of SEQ ID NO:7 to 14 and 19 to 22 are suitable for the detection and/or quantification of *E. coli,* whereas primer pairs and probes comprising nucleotide sequence of SEQ ID NO:15 to 18 and 23 to 24 are suitable for the detection and/or quantification of *Enterococcus spp.*

In another aspect, the present invention provides a method for detecting and/or quantifying living and viable fecal indicator bacteria in a water sample, comprising the steps of:
a) Providing a water sample,
b) Isolating ribonucleic acid (RNA) from said sample,
c) Performing a reverse transcription polymerase chain reaction (RT-PCR) amplification reaction with a mix comprising the isolated RNA, suitable PCR primer pairs capable of amplifying at least one nucleic acid target sequence selected from the group consisting of SEQ ID NO: 1 to SEQ ID NO:6,
d) Detecting and/or quantifying the RT-PCR amplifications products, wherein amplification of SEQ ID NO: 1, SEQ ID NO: 2, SEQ ID NO: 3 or SEQ ID NO: 4 is indicative of the presence of *E. coli* in said sample and wherein amplification of SEQ ID NO: 5 or SEQ ID NO: 6 is indicative of the presence of *Enterococcus spp.* in said sample.

In one embodiment, when the mix used in step c) of the method according to the invention also comprises suitable PCR primers capable of amplifying the nucleic acid target sequence of SEQ ID NO: 28, the method of the invention further comprises a step of detecting and/or quantifying *Staphylococcus aureus,* a strain also considered as a human source of water pollution.

In this particular embodiment, primer pair and probes comprising nucleotide sequence of SEQ ID NO: 25, SEQ ID NO: 26 and SEQ ID NO: 27 are suitable for the detection and/or quantification of *Staphylococcus aureus.*

The present invention also relates to amplicons obtained by amplification from fecal indicator bacteria-containing sample with primers of the invention, wherein said amplicon is bound to a probe of the invention, which comprise a fluorophore-quencher pair.

Finally, the present invention relates to kits for practicing the forgoing methods.

The present invention and its preferred embodiments are described in further detail below.

### DETAILED DESCRIPTION OF THE INVENTION

In a first aspect, the present invention relates to a method for detecting and/or quantifying living fecal indicator bacteria (FIB) in a water sample, comprising the steps of:
a) Providing a water sample,
b) Isolating ribonucleic acid (RNA) from said sample,
c) Performing a reverse transcription polymerase chain reaction (RT-PCR) amplification reaction with a mix comprising the isolated RNA, suitable PCR primer pairs capable of amplifying at least one nucleic acid target sequence selected from the group consisting of SEQ ID NO: 1, SEQ ID NO:2, SEQ ID NO: 3, SEQ ID NO:4, SEQ ID NO:5 and SEQ ID NO:6,
d) Detecting and/or quantifying the RT-PCR amplifications products, wherein amplification of SEQ ID NO: SEQ ID NO: 1, SEQ ID NO:2, SEQ ID NO: 3 or SEQ ID NO:4 is indicative of the presence of *E. coli* in said sample and wherein amplification of SEQ ID NO: SEQ ID NO:5 or SEQ ID NO:6 is indicative of the presence of *Enterococcus spp.* in said sample.

By "fecal indicator bacteria" (FIB) is meant nonpathogenic enteric bacteria, present at high concentrations in human and animal wastes, typically human and animal feces. FIB include fecal coliforms, *E. coli,* enterococci, or *Enterococcus spp.* Fecal indicator bacteria are commonly used to estimate human health risks in recreational waters internationally.

In order to deal with false-positive PCR results problem observed in prior art methods, the inventors developed a new reverse transcript (RT) PCR assay to distinguish between "living" and "dead" fecal indicator bacteria (FIB). By "living" FIB is meant bacteria in a state that they can proliferate, and also exhibit metabolic activities when cultured under appropriate conditions. By "dead" FIB is meant bacteria in a state that cannot proliferate and do not exhibit any metabolic activities. This new PCR assay is based on the use of RNA as a viability marker, on the basis of its rapid degradation compared to DNA. Thus, the method of the present invention is for detection and/or quantification of "living" fecal indicator bacteria (FIB).

In the present invention, the detection of living FIB includes both qualitative and quantitative determination in a water sample: qualitative determination when presence or absence of living FIB is determined in said water sample, quantitative determination when the amount of living FIB is determined in said water sample. The amount of living FIB is not limited to an absolute amount, and may be a relative amount with respect to that in a control sample.

Moreover, RNA as a viability marker can also be found in "viable" fecal indicator bacteria. By "viable" fecal indicator bacteria is meant fecal indicator bacteria that are in a state of very low metabolic activity (*i.e.* reduced nutrient transport, rate of respiration, and synthesis of macromolecules, ...) and do not divide, but are alive and have the ability to grow on standard solid or liquid growth media once external stress stops. Viable FIB can remain in that state for hours, months and sometimes for over a year.

External stress, such as adverse nutrient, temperature, osmotic, oxygen, and light conditions, can force bacteria to enter into a dormancy state wherein said bacteria - then referred to as dormant bacteria - have negligible metabolic activity. Thus, unlike living bacteria having the ability to proliferate, dormant bacteria are unable to divide and grow on standard media. Nevertheless, dormant bacteria are not considered as dying or dead bacteria since they are ultimately cultivable. Currently available assays based on colony counts may underestimate bacterial counts since colonies result solely from living bacteria.

The method of the invention provides unambiguous means of counting both living and viable bacteria.

As a consequence, the present invention relates to a method for detecting and/or quantifying living and viable fecal indicator bacteria (FIB) in a water sample, comprising the steps of:
a) Providing a water sample,
b) Isolating ribonucleic acid (RNA) from said sample,
c) Performing a reverse transcription polymerase chain reaction (RT-PCR) amplification reaction with a mix comprising the isolated RNA, suitable PCR primer pairs capable of amplifying at least one nucleic acid target sequence selected from the group consisting of SEQ ID NO: 1, SEQ ID NO:2, SEQ ID NO: 3, SEQ ID NO:4, SEQ ID NO:5 and SEQ ID NO:6,
d) Detecting and/or quantifying the RT-PCR amplifications products, wherein amplification of SEQ ID NO: SEQ ID NO: 1, SEQ ID NO: 2, SEQ ID NO: 3 or SEQ ID NO:4 is indicative of the presence of *E. coli* in said sample and wherein amplification of SEQ ID NO: SEQ ID NO:5 or SEQ ID NO:6 is indicative of the presence of *Enterococcus spp.* in said sample.

In all embodiments of the method of the invention, detection and/or quantification results of living and viable FIB are available in about 4 hours.

By "water sample" is meant water taken from ground water, spring water, surface water, sea water, waste water, seepage water, drinking water, groundwater bodies, springs, water distribution system, rivers, lakes, marine environments, sewage and waste water treatment systems. The water sample assessed by the method of the invention can be an individual or a pooled sample.

In all embodiments of the present invention, the water sample is suspected of containing the fecal indicator bacteria. This means that if at least one of fecal indicator bacteria is present in the water sample, it can be detected and/or quantified by the method of the invention.

According to a preferred embodiment, prior to performing RNA isolation, a step of preparing the sample may be carried out. The preparing step may comprise at least one of the following processes: (1) bacterial concentration in the sample, (2) separation of bacteria from the sample and (3) lysis of separated bacteria.

Preferably, the water sample provided in step a) is concentrated by filtration. Membranes 47 mm in diameter with a 0.45-µm pore size are typically used. However, alternate diameters and pore sizes can be employed provided that the pore size can sufficiently entrap the fecal indicator bacteria, and at least 70% of the filter area is comprised of pore space. The membrane filter used in the method of the invention can be, but are not limited to polycarbonate, polyvinylidene fluoride (PDVF), polytetrafluoroethylene (PFTE), mixed cellulose ester (MCE), polyethersulfone (PES) membrane filter. Preferably, the membrane filter used in the method of the invention is a PVDF membrane filter.

Preferably, the separation of bacterial cells from the sample is done by mechanical scrapping of the entrapped fecal bacterial indicator.

In one embodiment, the method according to the invention further comprises a step of filtrating and collecting bacteria prior to step b) of isolating RNA.

By "cell lysis" is intended "fecal indicator bacterial lysis". The aim of the step is to free the content of bacterial cells by disrupting the bacterial membrane. Physical methods such as sonication, freeze-thaw and liquid homogenization or solution-based methods using lysis buffer can be used. Both reagent-based methods and physical methods can be used to perform cell lysis. Preferably, the fecal indicator bacteria are disrupted by grinding using a lysis buffer.

By "isolating the ribonucleic acid (RNA)" is meant extracting the total ribonucleic acid including ribosomal RNA (rRNA), messenger RNA (mRNA) and transfer RNA (tRNA) from the whole nucleic acid content of the water sample. In this step, the extracted RNA may be purified in accordance with ordinary methods. The person skilled in the art is familiar with nucleic acid extraction methods and is able to select a suitable method depending in particular on the sample. There are no particular limitations on the methods used to extract and purify RNA from water sample, and a commercially available purification kit and the like can be used. In a more detailed process, the extraction step comprises the followings sub-steps: (1) nucleic acid extraction (*i.e.* both DNA and RNA) and (2) DNA elimination.

In one embodiment, the method according to the invention further comprises DNA elimination, so as to obtain pure RNA, prior to performing RT-PCR amplification reaction.

Furthermore, prior to proceeding to the next step, the concentration and amount of total RNA obtained in step b) may be measured using spectrophotometry, chips or any other known method in the relevant field.

By "RT-PCR amplification reaction" is meant the combination of both reverse transcription of RNA into cDNA and amplification of said cDNA into double stranded DNA. The reverse transcription can be achieved by a reverse transcriptase which uses an RNA template and a short primer complementary to the 3' end of the RNA to direct the synthesis of the first strand cDNA. Then the cDNA can be used directly as a template for the Polymerase Chain Reaction (PCR) wherein DNA polymerase amplifies DNA. By "amplification" is meant an increase of the concentration of a particular nucleic acid sequence, also called "target sequence", within a mixture of nucleic acid sequences. The term "target sequence" refers to the particular nucleotide sequence of the target nucleic acid which is to be amplified. RT-PCR can be performed in a one-step or a two-steps process. In a one-step process, cDNA synthesis and PCR amplification steps are performed in a single reaction using gene-specific primers, while in a two-step process, the synthetized cDNA can provide templates for multiple PCRs.

Preferably, the RT-PCR amplification reaction of step c) is a one-step process.

In one embodiment, the RT-PCR amplification reaction can be a real time RT-PCR, a digital RT-PCR or a droplet digital RT-PCR.

Amplification of all mRNA is avoided by using a specific primer for performing the reverse-transcription step of the RT-PCR amplification reaction.

In all embodiments of the method of the invention, reverse-transcription of RNA of target sequences uses the same reverse primers as those used for PCR amplification.

By "target sequence" is meant nucleic acid sequence comprising at least a gene or a portion of a gene from the genome of fecal indicator bacteria to be detected. The target sequence may be one of a single gene, or portion thereof, or two or more genes, or portions thereof. Specific examples of target sequences include, but are not limited to, rRNA and *uid* gene which codes for the 3-glucuronidase enzyme. Length of the target sequence is usually 50 to 250 nucleotides, preferably 60 to 220 nucleotides, and more preferably 80 to 200 nucleotides. The target sequence may be one containing a sequence specific to a particular category of FIB, such as a bacterial species or genus. Sequences of target sequences are listed in table 1.

**Table 1**

| **FIB** | **Target sequence** | **SEQ ID number** |
|---|---|---|
| *E. coli* | CATGTGGTTTAATTCGATGCAACGCGAAGAACC TTACCTGGTCTTGACATCCACGGAAGTTTTCAG AGATGAGAATGTGCCTTCGGGAA | SEQ ID NO: 1 |
| *E. coli* | CGGAAGCAACGCGTAAACTCGATCCGACGCGT CCGATCACCTGCGTCAATGTAATGTTCTGCGAC GC | SEQ ID NO: 2 |
| *E. coli* | GGCCACGGAGAGTTATCAGTTTGCTGCCCAGCA GCTGGTTCTGGGTATCCCTCAGGTGGCACTGCG TCCGCAGCTGAGGGGGATTGCGCCGGAGGCGT TATGGGATGATGGCATTCCGGCTTTTCTGCT | SEQ ID NO: 3 |
| *E. coli* | GCACTGTGACCACTCTGATTGCGCTGTGCCTGC CGGTGAGCGTTTTTGCCACCACGCTG | SEQ ID NO: 4 |
| *Enterococcus spp.* | GTTTCCGCCTTTCAGTGCTGCAGCTAACGCATTA AGCACTCCGCCTGGGGAGTACGACCGCAAGGT TGAAACTCAAAGGAATTGACGGGGGCCCGCAC AAGCGGTGGAGCATGTGGTTTAATTCGAAGCA ACGCGAAGAACCTTACCAGGTCTTGACATCCTT | SEQ ID NO: 5 |
| | TGACCACTCTAGAGATAGAGCTTCC | |
| *Enterococcus spp.* | GAGAAATTCCAAACGAACTTGGAGATAGCTGG TTCTCTCCGAAATAGCTTTAGGGCTAGCCTCGG AATTGAGAATGATGGAGGTAGAGCACTG | SEQ ID NO: 6 |

For example, when fecal indicator bacterium to be detected is *E. coli,* the target sequence is at least one sequence selected from the group consisting of SEQ ID NO:1, SEQ ID NO: 2, SEQ ID NO:3 and SEQ ID NO: 4. The target sequence for detecting the fecal indicator bacterium *Enterococcus spp.* is SEQ ID NO: 5 or SEQ ID NO: 6.

The number of amplified target sequences in the method according to the invention can be one, two, three, four, five or six.

By "suitable PCR primers" is meant oligonucleotides, whether occurring naturally or produced synthetically, enabling specific amplification of the aforementioned target sequence are chosen. Primers are capable of acting as a point of initiation of synthesis when placed under conditions in which synthesis of a primer extension product, which is complementary to a nucleic acid strand is induced, i.e. in the presence of nucleotides and a polymerization-inducing agent such as DNA polymerase and at a suitable temperature and in a suitable buffer. Such conditions include the presence in the mix of four different deoxyribonucleoside triphosphates (dNTPs) and a polymerization-inducing agent such as DNA polymerase or reverse transcriptase, in a suitable buffer ("buffer" includes agents which are cofactors, or affect pH, ionic strength, etc.), and at a suitable temperature.

The primer is preferably single-stranded for maximum efficiency in amplification. The primers herein are selected to be substantially complementary/identical to a strand of each specific sequence to be amplified. This means that the primers must be sufficiently complementary to hybridize with their respective strands. By "substantially complementary" or "substantially identical" is meant that the primers must be sufficiently complementary or identical to hybridize with their respective strands. As such, the primers sequence need not reflect the exact sequence of the template. Therefore, 85% or more, preferably 90% or more and most preferably 95% or more of nucleobases on one strand of the primer should be identical to the target sequence in an alignment such that the corresponding nucleotides can hybridize to each other.

In general, "identity" refers to an exact nucleotide-to-nucleotide correspondence of two polynucleotides or polypeptide sequences, respectively. Percent identity can be determined by a direct comparison of the sequence information between two molecules by aligning the sequences, counting the exact number of matches between the two aligned sequences, dividing by the length of the shorter sequence, and multiplying the result by 100. Readily available computer programs can be used to aid in the analysis, such as ALIGN, BLAST, EMBOSS Needle used with default parameters.

A "primer pairs" consists of a forward primer and a reverse primer. Primer pair refers to the forward and reverse primer in an amplification of a double-stranded DNA region of the target sequence. The forward primer is complementary to a first sequence of the target polynucleotide molecules and the reverse primer is complementary to a second sequence of the target polynucleotide molecules (the second sequence is on the opposite strand from the first sequence).

Specific examples of primer pairs suitable for amplifying a nucleic acid target sequence are listed in table 2.

**Table 2**

| **FIB** | **Primer sequence (5'→3')** | **SEQ ID number** |
|---|---|---|
| *E. coli* | CATGTGGTTTAATTCGATGCAAC | SEQ ID NO: 7 |
| | TTCCCGAAGGCACATTCTC | SEQ ID NO: 8 |
| *E. coli* | CGGAAGCAACGCGTAAACTC | SEQ ID NO: 9 |
| | GCGTCGCAGAACATTACATTGA | SEQ ID NO: 10 |
| *E. coli* | CGCCACGGAGAGTTATCAGTTT | SEQ ID NO: 11 |
| | AGCAGAAAAGCCGGAATGC | SEQ ID NO: 12 |
| *E. coli* | GCATCGTGACCACCTTGA | SEQ ID NO: 13 |
| | CAGCGTGGTGGCAAAA | SEQ ID NO: 14 |
| *Enterococcus spp.* | GTTTCCGCCCTTCA | SEQ ID NO: 15 |
| | GGAAGCTCTATCTCTAGAGTGG | SEQ ID NO: 16 |
| *Enterococcus spp.* | GAGAAATTCCAAACGAACTTG | SEQ ID NO: 17 |
| | CAGTGCTCACCTCCATCATT | SEQ ID NO: 18 |

Depending on the number of nucleic acid target sequences to amplify, the method according to the invention can be performed in a "simplex" or "multiplex" assay.

In a simplex assay, only one primer pair is used to amplify one target sequence whereas in a multiplex assay, at least two primer pairs are used to amplify more than one target sequence. In a multiplex assay, two, three, four, five, or more target sequences can be amplified. To avoid unspecific amplification in a multiplex assay, the designed primers should be checked for formation of primer dimers, with all the primers present in the reaction mix.

According to one embodiment, the method according to the invention is a simplex assay.

If primers specific to a particular fecal indicator bacterium are used, living cell of the particular FIB in the water sample can be detected. For example, the primer pairs of SEQ ID NO: 7 and 8, SEQ ID NO: 9 and 10, SEQ ID NO: 11 and 12 or SEQ ID NO: 13 and 14 are suitable for the detection of *E. coli* whereas the primers pairs of SEQ ID NO: 15 and 16 or SEQ ID NO: 17 and 18 are suitable for the detection of *Enterococcus spp.*

According to another embodiment, the method according to the invention is a multiplex assay.

If primers suitable for two or more fecal indicator bacteria are used, living cell of two or more FIB in the water sample can be detected. For example, the primer pairs of SEQ ID NO: 7 and 8 and SEQ ID NO: 15 and 16 are suitable for the amplification of the target sequences of the 16S rRNA gene of both *E. coli* and *Enterococcus spp.*

In a multiplex assay, the more than one amplified nucleic acid target sequence can be originated from only one FIB. For example, the primer pairs of SEQ ID NO: 7 and 8 and SEQ ID NO: 9 and 10, or the primer pairs of SEQ ID NO: 7 and 8 and SEQ ID NO: 11 and 12, or the primer pairs of SEQ ID NO: 7 and 8 and SEQ ID NO: 13 and 14, or the primer pairs SEQ ID NO: 9 and 10 and SEQ ID NO: 11 and 12, or the primer pairs SEQ ID NO: 9 and 10 and SEQ ID NO: 13 and 14, or the primer pairs SEQ ID NO: 11 and 12 and SEQ ID NO: 13 and 14, or the primer pairs of SEQ ID NO: 7 and 8 and SEQ ID NO: 9 and 10 and SEQ ID NO: 11 and 12, or the primer pairs of SEQ ID NO: 7 and 8 and SEQ ID NO: 9 and 10 and SEQ ID NO: 13 and 14, or the primers pairs of SEQ ID NO: 7 and 8 and SEQ ID NO: 11 and 12 and SEQ ID NO: 13 and 14, or the primer pairs of SEQ ID NO: 7 and 8 and SEQ ID NO: 9 and 10 and SEQ ID NO: 11 and 12 and SEQ ID NO: 13 and 14 can be used to amplify target sequence belonging to the genome of *E. coli* only. In another example, the primer pairs of SEQ ID NO: 15 and 16 and SEQ ID NO: 17 and 18 can be used to amplify target sequences belonging to the genome of *Enterococcus spp.*

Alternatively, in the "multiplex assay", the more than one amplified nucleic acid target sequence can be originated from two FIBs. For example, the primer pairs of SEQ ID NO: 7 and 8 and SEQ ID NO: 15 and 16, or the primer pairs of SEQ ID NO: 7 and 8 and SEQ ID NO: 17 and 18, or any other combination of primers pairs of SEQ ID NO: 7 to 14 and SEQ ID NO: 15 to 18 can be used to amplify target sequences belonging to the genome of *E. coli* and *Enterococcus spp..* In another example, the primer pairs of SEQ ID NO: 7 and 8 and SEQ ID NO: 15 and 16 and SEQ ID NO: 17 and 18 can be used to amplify target sequences belonging to the genome of *E. coli* and *Enterococcus spp..*

Complementary sequence of the primer sequence recited in table 2 can also be used in step c) of the method according to the invention. The "complement of a nucleic acid sequence" refers in the present invention to an oligonucleotide which, when aligned with the nucleic acid sequence such that the 5' end of one sequence is paired with the 3' end of the other, and is in "antiparallel association". Complementarity need not be perfect; stable duplexes may contain mismatched base pairs or unmatched bases.

Polynucleotides derived from the primer sequence recited in table 2 can also be used in step c) of the method according to the invention. Polynucleotides "derived from" a designated sequence refers in the present invention to a polynucleotide sequence which comprises a contiguous sequence of approximately at least about 13 nucleotides, preferably at least about 15 nucleotides, more preferably at least about 17-18 nucleotides, more preferably at least about 20-21 nucleotides, and even more preferably at least about 19-22 nucleotides corresponding, i.e., identical or complementary to, a region of the designated nucleotide sequence. The derived primer will not necessarily be derived physically from the nucleotide sequence of interest, but may be generated in any manner, including, but not limited to, chemical synthesis, replication, reverse transcription or transcription, which is based on the information provided by the sequence of bases in the region(s) from which the primer is derived. As such, it may represent either a sense or an antisense orientation of the original polynucleotide.

In one embodiment, the sequence of the primer used for reverse-transcribing RNA from *E. coli* into cDNA is SEQ ID NO: 8, SEQ ID NO: 10, SEQ ID NO: 12 or SEQ ID NO: 14, preferably SEQ ID NO: 8, SEQ ID NO: 10 or SEQ ID NO: 12, and more preferably SEQ ID NO:8.

In one embodiment, the sequence of the primer used for reverse-transcribing RNA from *Enterococcus spp.* into cDNA is SEQ ID NO: 16 or SEQ ID NO: 18, preferably SEQ ID NO: 16.

The mix used for performing the RT-PCR refers to a mixture of components necessary to amplify at least on target sequence from RNA template(s). The mix may comprises deoxyribonucleotide triphosphates (dNTPs), thermostable RNA-dependent reverse transcriptase and DNA polymerase (or a thermostable DNA polymerase with intrinsic reverse transcriptase activity), primers, a plurality of RNA template(s), Tris buffer, a monovalent salt and Mg²⁺. The concentration of each component is well known in the art and can be further optimized by the skilled artisan.

The mix used in step c) of the method according to the invention can also comprise "labelled oligonucleotides", or "probes", both terms being interchangeable. By "probe" is meant an oligonucleotide that is capable of forming a duplex structure by complementarity base pairing with a sequence of the target sequence.

Specific examples of probes suitable for hybridizing a nucleic acid target sequence are listed in table 3.

**Table 3**

| **FIB** | **Probe sequence (5'→3')** | **SEQ ID number** |
|---|---|---|
| *E. coli* | CATCCACGGAAGTTTTC (or CATCCACGGAAGTTT*T*C) | SEQ ID NO: 19 |
| *E. coli* | ACCCGACGCGTCCGATCACCT | SEQ ID NO: 20 |
| *E. coli* | TATCCCTCAGGTGGCACTGCGTCC | SEQ ID NO: 21 |
| *E. coli* | TGCATTATGTTTGCCGGTATCCG | SEQ ID NO: 22 |
| *Enterococcus spp.* | GACATCCTTTGACCAC (or GACATCCTTTGACCA*C) | SEQ ID NO: 23 |
| *Enterococcus spp.* | TGGTTCTCTCCGAAATAGCTTTAG GGCTA | SEQ ID NO: 24 |

In Table 3, the nucleotides followed by an * are superbases. Such nucleotides are modified to have an additional hydrogen bond with the complementary base. These superbases allow stabilizing the matching.

Particularly crucial for primers and probes design is having an appropriate melting temperature (Tm), which determines the conditions under which these will bind to a target sequence. The "melting temperature" or "Tm" of double-stranded DNA is defined as the temperature at which half of the helical structure of DNA is lost due to heating or other dissociation of the hydrogen bonding between base pairs, for example, by acid or alkali treatment, or the like. The Tm of a DNA molecule depends on its length and on its base composition. DNA molecules rich in GC base pairs have a higher Tm than those having an abundance of AT base pairs. Separated complementary strands of DNA spontaneously reassociate or anneal to form duplex DNA when the temperature is lowered below the Tm. The highest rate of nucleic acid hybridization occurs approximately 25 degrees C below the Tm. The Tm may be estimated, for example, using the following relationship: Tm= 69.3 + 0.41(GC)%.

In another aspect, the method for detecting and/or quantifying viable fecal indicator bacteria in a sample, comprises the steps of:
a) providing a water sample,
b) isolating ribonucleic acid (RNA) from said sample,
c) subjecting the isolated RNA to reverse-transcription real time PCR using at least one primer pair comprising two oligonucleotides selected in table 2, and optionally at least one probe selected in table 3, and
d) detecting and/or quantifying the resulting RT-PCR amplifications products.

Primer and probes used in the method of the invention can be natural nucleic acid or Peptide Nucleic Acid (PNA) which can hybridize to DNA and RNA nucleic acid. Peptide nucleic acids (PNAs) are synthetic homologs of nucleic acids in which the phosphate-sugar polynucleotide backbone is replaced by a flexible pseudo-peptide polymer to which the nucleobases are linked. This structure gives PNAs the capacity to hybridize with high affinity and specificity to complementary sequences of DNA and RNA, and also confers remarkable resistance to DNAses and proteinases.

Generally, a probe is labeled with a reporter dye at its 5' end and a quencher dye at its 3' end.

By "reporter dye" is meant a moiety that absorbs light energy at a defined excitation wavelength and emits light energy at a different defined wavelength.

Examples of reporter dye include, but are not limited to, FAM™(carboxyfluorescein), TET™(tetrachlorofluorescein), HEX™(hexachlorofluorescein), Cy5® (1-(e-carboxypentyl)- 1'-ethyl-3, 3,3', 3'-tetramethy 1-5, 5'-disulphonato- dicarbocyanine), Cy5.5® (1-(e-carboxypentyl)-1'-ethyl-3, 3,3',3'-tetramethyl-4, 5,4', 5'-(1,3-disulphonato)- dibenzo-dicarbocyanine), Cy3® (3-(e-carboxypentyl)-1'- ethyl-3, 3,3',3'-tetramethyl-5, 5'-disulphonato-carbocyanine), Cy3.5® (3-(e-carboxypentyl)-1'-ethyl-3, 3,3', 3'-tetramethyl- 4,5,4', 5'-(1,3-disulphonato)dibenzo-carbocyanine), Cy7® (1-(e-carboxypentyl)- 1'-ethyl-3, 3,3",3'-tetramethy1-5, 5'-di sulphonato- tricarbocyanine), TAMRA™(carboxytetramethylrhodamine), Texas Red®, ROX™(carboxy-X-rhodamine), JOE™ (6-carboxy-4', 5'-dichloro-2', 7'-dimethoxyfluorescein), ATTO™ dyes and TYE™ dyes.

Preferably, the reporter dye of the probe used in the method according to the invention is selected from the group consisting of FAM™, Cy5®, JOE™ and HEX™.

When used in a multiplex assay, probes may be labelled with different dyes so that they can be detected in different detection channels.

By "quencher" is meant a moiety that is capable of absorbing the energy of an excited fluorescent dye when it is located in close proximity to the fluorescent dye and capable of dissipating that energy. A quencher can be a fluorescent quencher or a non-fluorescent quencher, which is also called dark quencher. Because they do not emit any visible light, dark quenchers are preferred.

Examples of dark quencher include, but are not limited to, DABCYL (4-(4'-dimethylaminophenylazo) benzoic acid) succinimidyl ester, diarylrhodamine carboxylic acid, succinimidyl ester (QSY-7), and 4',5'-dinitrofluorescein carboxylic acid, succinimidyl ester (QSY-33), or "Black hole quenchers" (BHQ-1, BHQ-2 and BHQ-3), non-fluorescent quencher (NFQ), nucleotide analogs, nucleotide G residues, nanoparticles, and gold particles.

Preferably, the quencher of the probe used in the method according to the invention is selected from the group consisting of BHQ1, BHQ2 and NFQ.

In order to facilitate or enhance probe binding, chemical modifications can be introduced at the 3' end of the probe, such as minor groove binders (MGBs) or Zip nucleic acids (ZNAs).

MGBs are chemical moieties that can adopt a crescent shape allowing them to bind to duplex DNA in the minor groove. MGBs are stabilized in the minor groove by either hydrogen bonds or hydrophobic interactions, resulting in the stabilization of the probe-target sequence hybrids.

ZNAs are oligonucleotides conjugated with repeated cationic spermine units that decrease electrostatic repulsions with target nucleic acid strands. The number of cationic units attached at any position of the oligonucleotide modulates the global charge of the molecule, thus raising the corresponding duplex Tm in a smooth, linear and predictable manner.

Preferably, the probe used in the method according to the invention is chemically modified at the 3' end with either MGBs or ZNAs.

Fluorophore-quencher pairs that have sufficient spectral overlap should be chosen. Fluorescent dyes with an emission maximum between 500 and 550 nm, such as FAM™, TET™ and HEX™ are best quenched by quenchers with absorption maxima between 450 and 550 nm, such as DABCYL and BHQ-1. Fluorophores with an emission maximum above 550 nm, such as Rhodamines (including TAMRA™, ROX™ and Texas Red®) and Cy® dyes are best quenched by quenchers with absorption maxima above 550 nm (including BHQ2).

Fluorophore-labeled probe has been divided into two subgroups according to the type of fluorescent molecules used in the PCR reaction: (i) primer-probes (Scorpions, Amplifluor, LUX, Cyclicons, Angler) and (ii) probes; hydrolysis (TaqMan, MGB-TaqMan, Snake assay) and hybridization (Hybprobe or FRET, Molecular Beacons, HyBeacon, MGB-Pleiades, MGB-Eclipse, ResonSense, Yin-Yang or displacing).

Preferably, the probe used in the method according to the invention is TaqMan® or MGB-TaqMan® probe.

TaqMan® probes derive their fluorescence signal from the hydrolysis of the probe by DNA polymerase's 5' to 3' exonuclease activity. During amplification, when the polymerase replicates a template on which a TaqMan® probe is bound, the 5' exonuclease activity of the polymerase cleaves the probe. Fluorescence can be read. Fluorescence increases in each cycle, proportional to the rate of probe cleavage. TaqMan® and MGB-TaqMan®, both of which are hydrolysis probes accumulation for quantification.

In the method of the invention, FAM™ dye is preferably associated with a quencher selected in the group consisting of BHQ1, MGB-NFQ and ZNA-4-BHQ1, Cy5® is preferably associated with BHQ2 quencher, JOE™ and HEX™ dies are generally associated with BHQ1 quencher.

In one embodiment, the sequences of primers and probes used for the amplification of cDNA from E.coli are SEQ ID NO: 7 + SEQ ID NO: 8 + SEQ ID NO: 19, or SEQ ID NO: 9 + SEQ ID NO: 10 + SEQ ID NO: 20, or SEQ ID NO: 11 + SEQ ID NO: 12 + SEQ ID NO: 21, or SEQ ID NO: 13 + SEQ ID NO: 14 + SEQ ID NO: 22. Preferably, the sequences of primers and probes used for the amplification of cDNA from E.coli are SEQ ID NO: 7 + SEQ ID NO: 8 +SEQ ID NO: 19.

In one embodiment, the sequences of primers and probes used for the amplification of cDNA from *Enterococcus spp.* are SEQ ID NO: 15 + SEQ ID NO: 16 + SEQ ID NO: 23, or SEQ ID NO: 17 + SEQ ID NO: 18 + SEQ ID NO: 24. Preferably, the sequences of primers and probes used for the amplification of cDNA from *Enterococcus spp.* are SEQ ID NO: 15 + SEQ ID NO: 16 + SEQ ID NO: 23.

In a multiplex assay, the preferred combination of primers and probes for the amplification of cDNA from both E.coli and *Enterococcus spp.* are SEQ ID NO: 7 + SEQ ID NO: 8 + SEQ ID NO: 19 and SEQ ID NO: 15 + SEQ ID NO: 16 + SEQ ID NO: 23, or SEQ ID NO: 7 + SEQ ID NO: 8 + SEQ ID NO: 19 and SEQ ID NO: 17 + SEQ ID NO: 18 + SEQ ID NO: 24.

Along with fecal indicator bacteria, also potentially pathogenic bacteria whose normal habitat is not recreational water environments are introduced into the recreational water. Examples of possible pathogenic bacteria include, but are not limited to, *Aeromonashydrophila, Campylobacter spp., Clostridium perfringens, Klebsiella pneumoniae, Neisseria spp., Pseudomanas aeruginosa, Salmonella spp., Shigella spp., Staphylococcus aureus* and *Vibrio cholerae.* The accumulation of these and other pathogenic bacteria in the recreational water represents a hazard to bathers, i.e. leads to a potential disease.

In particular, bathers shed large numbers of pathogenic bacteria, mainly *Staphylococcus aureus,* via their skin into the water column, and swimming-related diseases appear to be associated with the microbial water quality, even in the absence of the point source of bacterial contamination.

According to a preferred embodiment, the method according to the invention further comprises a step of detecting and/or quantifying *Staphylococcus aureus.*

The primers and probes useful for the detection and/or quantification of *Staphylococcus aureus* are listed in table 4.

**Table 4**

| **Name** | **Primer /Probe sequence (5'→3')** | **SEQ ID number** |
|---|---|---|
| Forward primer | GGTTCAAAAGTGAAAGACGGTC | SEQ ID NO: 25 |
| Reverse primer | CCTCTCAGGTCGGCTATGC | SEQ ID NO: 26 |
| Probe | GGATCCGCGCTGCATTAG | SEQ ID NO: 27 |
| Target sequence | GGTTCAAAAGTGAAAGACGGTCT TGCTGTCACTTATAGATGGATCCG CGCTGCATTAGCTAGTTGGTAAG GTAACGGCTTACCAAGGCAACGA TGCATAGCCGACCTGAGAGG | SEQ ID NO: 28 |

By adding the primer pair of SEQ ID NO: 25 and SEQ ID NO: 26 as recited in table 4 in the mix used in step c) of the method of the invention, one can amplify the nucleic acid target of SEQ ID NO: 28.

By adding the probe of SEQ ID NO: 27 as recited in table 4 in the mix used in step c) of the method of the invention, one can detect the nucleic acid target of SEQ ID NO: 28.

In one embodiment, the method of the invention further comprises a step of detecting and/or quantifying *Staphylococcus aureus,* wherein the mix comprising the isolated RNA also comprises suitable PCR primers capable of amplifying a nucleic acid target sequence of SEQ ID NO: 28 and wherein amplification of SEQ ID NO: 28 is indicative of the presence of *Staphylococcus aureus* in said sample.

Complementary sequence of the primer sequence recited in table 4 can also be used in step c) of the method according to the invention. The "complement of a nucleic acid sequence" refers in the present invention to an oligonucleotide which, when aligned with the nucleic acid sequence such that the 5' end of one sequence is paired with the 3' end of the other, and is in "antiparallel association". Complementarity need not be perfect; stable duplexes may contain mismatched base pairs or unmatched bases. Polynucleotides derived from the primer sequence recited in table 4 can also be used in step c) of the method according to the invention. Polynucleotides "derived from" a designated sequence refers in the present invention to a polynucleotide sequence which comprises a contiguous sequence of approximately at least about 18 nucleotides, more preferably at least about 21 nucleotides and more preferably at least about 18-21 nucleotides corresponding, i.e., identical or complementary to, a region of the designated nucleotide sequence. The derived primer will not necessarily be derived physically from the nucleotide sequence of interest, but may be generated in any manner, including, but not limited to, chemical synthesis, replication, reverse transcription or transcription, which is based on the information provided by the sequence of bases in the region(s) from which the primer is derived. As such, it may represent either a sense or an antisense orientation of the original polynucleotide.

It is of importance to distinguish false negative results from true ones in highly sensitive techniques such as RT-PCR. Several factors can generate a false negative result, such as errors in sample extraction or thermocycler malfunction. RT-PCR inhibition is the most common cause of assay failure. The most practical approach to control the presence of inhibitors is to include an Internal Positive Control, or Internal Control (IC). This IC is simultaneously extracted and amplified (or only amplified) in the same tube with the nucleic acid target sequence of FIB. The use of IC enables confirming that a negative result is truly negative.

The mix used in step c) of the method according to the invention can also comprises a pair of control primers to produce a synthetic internal control gene amplification product, wherein the pair of control primers amplifies a synthetic internal control gene segment that is not a target sequence of FIB, and a labelled probe that specifically hybridizes to the synthetic internal control gene amplification products.

In the method according to the invention, the sequences of primers, probe and target sequence are listed in table 5.

**Table 5**

| **Name** | **Primer /Probe sequence (5'→3')** | **SEQ ID number** |
|---|---|---|
| Forward primer | GCAATCGTATTACCTCTTATCGCAG | SEQ ID NO: 29 |
| Reverse primer | CAACCATCGTCATCGTCAGGAAAC | SEQ ID NO: 30 |
| Probe | GGCAAAGTCCCATCGTT | SEQ ID NO: 31 |
| Target sequence | GCAATCGTATTACCTCTTATCGCAG AAAAGGCAAAGTCCCATCGTTAAA AGTTTCCTGACGATGACGATGGTT G | SEQ ID NO: 32 |

For example, the primer pairs of SEQ ID NO: 7 and 8 and SEQ ID NO: 29 and 30 can be used to amplify target sequences belonging to the genome of *E. coli* and internal control gene amplification product.

At the end of the RT-PCT amplification step, the amplification products are analyzed. By "amplification product" is meant the double stranded DNA sequence resulting of the amplification step using at least suitable (i.e. specific) primers of the target sequence. The amplification products are also called amplicons. The two terms amplification product and amplicon have the same meaning and can be used interchangeably.

By "detecting the RT-PCR amplification products" is meant assessing the presence of the absence of the expected amplification products. Presence or absence of the amplification products can be determined by analyzing the melting temperature (TM) pattern of the amplification products or by electrophoresis wherein amount and size of the amplification products can be evaluated.

There are two general approaches to amplicon detection, specific and non-specific fluorescent reporting chemistries. The specific one uses specific probes as previously disclosed. The non-specific fluorescent reporting chemistry uses intercalating dyes such as SYBR® Green Dye, which binds to the minor groove of the DNA double helix. In solution, the unbound dye exhibits very little fluorescence, however, fluorescence is greatly enhanced upon DNA-binding. When the reaction of amplification is monitored continuously, an increase in fluorescence is viewed in real-time.

Moreover, when SYBR® Green Dye or labelled probes were present in the mix used in step c) of the method of the invention, the amplification products can also be quantified by real-time PCR or real-time RT-PCR (also named RT-qPCR, qRT-PCR), digital RT-PCR, or droplet digital RT-PCR.

Amplification products are quantified as they accumulate, as opposed to performing a fixed number of cycles and then assessing the amount and variety of products by electrophoresis. There are two ways to approach data analysis in real-time PCR applications. The first is the relative quantification method, whereby the amplification of an experimental template is compared to that of a control sample. The other method, known as absolute quantification, is used to calculate the actual copy number of template molecules with statistical confidence. It involves comparing the amplification behavior of an experimental sample against a standard curve and is sometimes referred to as the standard curve method.

Quantification may be achieved by comparing the amplification product to an external standard curve representing relationship of amount of the microorganism and the amplification product, prepared by using standard samples of the microorganism.

In one embodiment, the RT-PCR amplification product obtained at the end of step c) of the method according to the invention is analyzed by using a standard curve presenting a relationship between amount of the living fecal indicators bacteria and the amplification product, prepared by using standard samples of fecal indicator bacteria.

Calibrated microbial references containing a precise number of microorganisms for different strains of FIB are commercially available. Such references may also be used as an external positive control to prove the functionality of the reaction mix for amplification of the nucleic acid target sequence of FIB.

In all embodiments, the method of the invention can be carried out on a sample containing a population of *E. coli* from 10 to 10⁷ CFU/100 ml, preferably from 250 to 2.5.10⁵ CFU/100 ml.

In all embodiments, the method of the invention can be carried out on a sample containing a population of *E. coli* from 10 to 10⁷ copies/ml, preferably from 100 to 10⁵ copies/ml.

In all embodiments, the method of the invention can be carried out on a sample containing a population of *Enterococcus spp.* from 10 to 10⁷ CFU/100 ml, preferably from 10 to 10⁴ CFU/100 ml.

In all embodiments, the method of the invention can be carried out on a sample containing a population of *Enterococcus spp.* from 10 to 10⁷ copies/ml, preferably from 100 to 10⁵ copies/ml.

In some embodiments, the method of the invention can be carried out on a sample containing a population of *Staphylococcus aureus* from 10 to 10⁷ CFU/100 ml, preferably from 1 to 2.10⁴ CFU/100 ml.

In all embodiments, the method of the invention can be carried out on a sample containing a population of *Staphylococcus aureus* from 10 to 10⁷ copies/ml, preferably from 100 to 10⁵ copies/ml.

Further methods for quantification are well known in the art and include for example spiking, direct calculation from threshold data and serial dilution.

In a second aspect, the present invention relates to primer pairs and sets of primers suitable for the detection of nucleic acid target sequence from fecal indicator bacteria.

As a consequence, the present invention relates to primer set for amplifying a fecal indicator bacteria polynucleotide comprising one or more primer pair selected from the group consisting of SEQ ID NO: 7 and 8, SEQ ID NO: 9 and 10, SEQ ID NO: 11 and 12, SEQ ID NO: 13 and 14, SEQ ID NO: 15 and 16, and SEQ ID NO: 17 and 18.

By "primer set" is meant at least one primer pair comprising one forward primer and one reverse primer. The primer set can thus comprises one, two, three, four, five or more primer pairs suitable for amplifying at least one nucleic acid target sequence of a FIB as disclosed above. The primer set may further comprise at least one primer pair suitable for the amplification of a nucleic acid target sequence of from *Staphylococcus aureus.* In another embodiment, the primer set comprises a primer pair suitable for the amplification of a synthetic internal control gene segment. Finally, the primer set may further comprise at least one probe for detecting at least one amplicon obtained by the methods of the invention.

In one embodiment, the primer pair comprises a forward primer selected from the group consisting of SEQ ID NO: 7, SEQ ID NO: 9, SEQ ID NO: 11 and SEQ ID NO: 13, and a reverse primer selected from the group consisting of SEQ ID NO: 8, SEQ ID 10, SEQ ID NO: 12 and SEQ ID NO: 14.

In one embodiment, the primer set may further comprise a probe selected from the group consisting of SEQ ID NO: 19, SEQ ID NO:20, SEQ ID NO:21 and SEQ ID NO: 22.

In another embodiment, the primer pair comprises a forward primer selected from the group consisting of SEQ ID NO: 15 and SEQ ID NO: 17, and a reverse primer selected from the group consisting of SEQ ID NO: 16 and SEQ ID NO: 18.

In another embodiment, the set of primer may further comprise a probe selected from the group consisting of SEQ ID NO: 23 and SEQ ID NO: 24.

In still another embodiment, the primer pair comprises a forward primer of SEQ ID NO: 25, and a reverse primer of SEQ ID NO: 26.

In another embodiment, the set of primer may further comprise a probe of SEQ ID NO: 27.

In still another embodiment, the primer pair comprises a forward primer of SEQ ID NO: 29, and a reverse primer of SEQ ID NO: 30.

In another embodiment, the set of primer may further comprise a probe of SEQ ID NO: 31.

In a third aspect, the present invention relates to an amplicon obtainable by amplification from fecal indicator bacteria-containing water sample with a forward primer of SEQ ID NO: 7 and a reverse primer of SEQ ID NO: 8, wherein said amplicon is bound to a probe comprising a fluorophore-quencher pair, wherein said probe is of SEQ ID NO: 19.

In one embodiment, the amplicon is obtained by amplification from fecal indicator bacteria-containing sample with a forward primer of SEQ ID NO: 9 and a reverse primer of SEQ ID NO: 10, wherein said amplicon is bound to a probe comprising a fluorophore-quencher pair, wherein said probe is of SEQ ID NO: 20.

In one embodiment, the amplicon is obtained by amplification from fecal indicator bacteria-containing sample with a forward primer of SEQ ID NO: 11 and a reverse primer of SEQ ID NO: 12, wherein said amplicon is bound to a probe comprising a fluorophore-quencher pair, wherein said probe is of SEQ ID NO: 21.

In one embodiment, the amplicon is obtained by amplification from fecal indicator bacteria-containing sample with a forward primer of SEQ ID NO: 13 and a reverse primer of SEQ ID NO: 14, wherein said amplicon is bound to a probe comprising a fluorophore-quencher pair, wherein said probe is of SEQ ID NO: 22.

In one embodiment, the amplicon is obtained by amplification from fecal indicator bacteria-containing sample with a forward primer of SEQ ID NO: 15 and a reverse primer of SEQ ID NO: 16, wherein said amplicon is bound to a probe comprising a fluorophore-quencher pair, wherein said probe is of SEQ ID NO: 23.

In one embodiment, the amplicon is obtained by amplification from fecal indicator bacteria-containing sample with a forward primer of SEQ ID NO: 17 and a reverse primer of SEQ ID NO: 18, wherein said amplicon is bound to a probe comprising a fluorophore-quencher pair, wherein said probe is of SEQ ID NO: 24.

In one embodiment, the amplicon is obtained by amplification from *Staphylococcus aureus*-containing sample with a forward primer of SEQ ID NO: 25 and a reverse primer of SEQ ID NO: 26, wherein said amplicon is bound to a probe comprising a fluorophore-quencher pair, wherein said probe is of SEQ ID NO: 27.

The invention also provides a kit suitable for the implementation of the methods according to the invention.

In a fourth aspect, the present invention relates to a kit for the detection and/or quantification of fecal indicator bacteria (FIB) in a water sample, comprising means for the detection of at least one target sequence of SEQ ID NO: 1, SEQ ID NO: 2, SEQ ID NO: 3, SEQ ID NO: 4, SEQ ID NO: 5 or SEQ ID NO: 6 and instructions how to use the kit. By "means for the detection and/or amplification" is meant at least one primer pair, and optionally at least one probe suitable for the detection of at least one target sequence as disclosed previously.

In one embodiment, the means for the detection of at least one target sequence of SEQ ID NO: 1, SEQ ID NO: 2, SEQ ID NO: 3, SEQ ID NO: 4 comprise at least one primer pair suitable for amplifying at least one target sequence of SEQ ID NO: 1, SEQ ID NO: 2, SEQ ID NO: 3 or SEQ ID NO: 4, and optionally at least one probe for quantifying the said at least one target sequence, wherein the at least one primer pair is selected from the group consisting of SEQ ID NO: 7 and 8, SEQ ID NO: 9 and 10, SEQ ID NO: 11 and 12 and SEQ ID NO: 13 and 14, and wherein the at least one probe is selected from the group consisting of SEQ ID NO: 19, SEQ ID NO: 20, SEQ ID NO: 21 and SEQ ID NO: 22.

All these primer pairs and probe are suitable for the detection of *E. coli.* More particularly, the primer pairs and probes are suitable for the amplification of at least one nucleic acid target sequence of SEQ ID NO: 1, SEQ ID NO: 2, SEQ ID NO: 3 or SEQ ID NO: 4.

In another embodiment, the means for the detection of at least one target sequence of SEQ ID NO: 5 or SEQ ID NO: 6 comprises at least one primer set suitable for amplifying at least one target sequence of SEQ ID NO: 5 or SEQ ID NO: 6, and optionally at least one probe for quantifying the said target sequence, wherein the at least one primer set is selected from the group consisting of SEQ ID NO: 15 and 16 and SEQ ID NO: 17 and 18, and wherein the at least one probe is selected from the group consisting of SEQ ID NO: 23 and SEQ ID NO: 24.

All these primer pairs and probes are suitable for the detection of *Enterococcus spp.* More particularly, the primer pairs and probes are suitable for the amplification of at least one nucleic acid target sequence of SEQ ID NO: 5 or SEQ ID NO: 6.

When the kit is used in a multiplex assay format, the kit comprises primer pairs and probes suitable for the detection and quantification of both *E. coli* and/or *Enterococcus spp.*

In one embodiment, the means for the detection of at least one target sequence of SEQ ID NO: 1, SEQ ID NO: 2, SEQ ID NO: 3, SEQ ID NO: 4, SEQ ID NO: 5 or SEQ ID NO: 6 comprise at least one primer set suitable for amplifying at least one target sequence of SEQ ID NO: 1, SEQ ID NO: 2, SEQ ID NO: 3, SEQ ID NO: 4, SEQ ID NO: 5 or SEQ ID NO:6, and optionally at least one probe for quantifying the said target sequence, wherein the at least one primer set is selected from the group consisting of SEQ ID NO: 7 and 8, SEQ ID NO: 9 and 10, SEQ ID NO: 11 and 12, SEQ ID NO: 13 and 14, SEQ ID NO: 15 and 16 and SEQ ID NO: 17 and 18, and wherein the at least one probe is selected from the group consisting of SEQ ID NO: 19, SEQ ID NO: 20, SEQ ID NO: 21, SEQ ID NO: 22 and SEQ ID NO: 23 and SEQ ID NO: 24.

If *Staphylococcus aureus* is also to be detected and/or quantified, the kit further comprises at least one primer pair suitable for the amplifying at least one target sequence of SEQ ID NO: 28, and optionally at least one probe for quantifying the said target sequence, wherein the at least one primer set is SEQ ID NO: 25 and 26, and wherein the at least one probe is SEQ ID NO: 27.

In all embodiments, the kit may further comprise primer pair of SEQ ID NO: 29 and SEQ ID NO: 30 suitable for amplifying a target sequence of the internal positive control of SEQ ID NO: 32, and optionally the probe of SEQ ID NO: 31 for quantifying said target sequence.

Moreover, in all embodiments, the kit comprises a mix for RT-PCR amplification comprising all the buffers, intercalating dye such as SYBR® Green and components commonly used in RT-PCR amplification assay. The kit can also contain a control sample, a standard, or a series of control samples or standard that are assayed and compared to the test sample. Each component of the kit is optionally enclosed within an individual container or vial and all the various containers or vials are optionally enclosed within a single package along with instructions of use.

Moreover, in all embodiments, the kit may further comprise a notice of instructions for use of said primers and probes for the detection and/or quantification of fecal indicator bacteria.

The following examples are provided to illustrate certain preferred embodiments and aspects of the present invention and are not to be construed as limiting the scope thereof.

### EXAMPLES

### 1. Sample collection

Water monitoring samples were collected from the field at sites with suspected sources of contamination. From 100 to 5000 ml of water samples were collected in bottles, transported and stored at 4 degrees centigrade until laboratory processing (within 4 hours of collection).

When water monitoring sample is turbid or expected to contain high concentrations of fecal indicator bacteria, a smaller volume can be collected.

### 2. Sample processing

100 ml of thoroughly mixed water sample was passed through a filter unit containing a 47 mm, 0.45 µm pore size (PVDF) filter. The filter was then rinsed in a small volume of lysis buffer containing guanidine thiocyanate and the bacteria were collected by scraping the PVDF filter. The lysis buffer containing the collected bacteria was placed in 2 ml screw cap tube containing from 400 mg to 1000 mg of 500 µm acid-washed glass beads (BlueDNAcompanion,France) and processed for cell disruption.

The bead beating protocol was performed with a vortex for 10 minutes or using a homogenizer like Precellys® or Minilys® (Bertin INSTRUMENTS, France). The grinding protocol was 3 mixing steps during 45 sec with a speed between 5000 and 6500 RPM with a 30 seconds pause step between each mixing steps. The supernatant, containing the intracellular content of the lysed bacteria, is retained for nucleic acid extraction and the pellet is discarded.

### 3. RNA extraction

RNA extraction was done manually or using a paramagnetic silica beads system such as GenoSPYD IDEAL®, KingFisher® Flex 96 or KingFisher® mL instrument, BlueDNAcompanion kits (MagSPYD Total RNA kit) and consumables according to manufacturer's protocols (BlueDNAcompanion, France or Thermo Fisher Scientific, Finland): The principle of the extraction system is to transfer the paramagnetic silica beads in different appropriate buffers using metallic tips. Briefly, the following steps are done:
- Step 1: Nucleic acids binding on silica magnetic beads was obtained as follows: 500 µl of binding buffer and 750 µl to 1000 µl supernatant were added to 25 µl of paramagnetic beads under appropriate incubation condition : at least 5 min at room temperature under mixing;
- Step 2: DNA elimination was done by transferring paramagnetic silica beads into 300 µl of rDNase working solution to degrade DNA for at least 10 min at 37°C under mixing:
- Step 3: rDNase elimination was done by transferring paramagnetic silica beads into up to 850 µl of buffer containing chaotropic salt, such as guanidiniumisothiocyanate, to degrade proteins including DNase, for at least 5 min at room temperature under mixing;
- Step 4: Chaotropic salt was eliminated by transferring paramagnetic silica beads into up to 850 µl of buffer containing ethanol for at least 5 min at room temperature under mixing. This step can be repeated twice;
- Step 5: Ethanol was removed by drying beads at room temperature;
- Step 6: Pure RNAs were elutedusing up to 500 µl of elution buffer for at least 5 min at 70°C.

Total RNA concentrations for each sample can be estimated directly with Thermo Scientific NanoDrop without diluting the sample or by measuring its absorbance at 320 nm, 280 nm, and 260 nm. The ratio between the A260/A280 indicates the purity of the RNA. Concentration of RNA can be calculated using the formula: 40 µg/ml x (A260 - A320) x dilution factor.

### 4. Genomic calibration

A RNA calibration standards were prepared from calibrated bioballs® provided by Biomérieux, France. For *Escherichia coli,* Bioball® E.coli, strain NCTC 12923, Ref 413833 at 30.4 CFU ±2.1 and Ref 56006 at 576 CFU ±42.3 were used. For *Enterococcus spp.,* Bioball® Enterococcus, strain NCTC 12697, Ref 560053 at 566.9CFU ±28.7 was used. Extraction of RNAs from standards was carried out according to the same protocol as that used for water samples.

### 5. qRT-PCR

A reaction mixture was prepared comprising dNTPs, manganese acetate (or suitable cation), reaction buffer, enzyme with both DNA polymerase and reverse transcriptase activity like Tth manufactured by BlueDNAcompanion, primers and probes suitable for the amplification and detection of target sequence belonging to the genome of *E. coli* and/or *Enterococcus spp.* and optionally primers and probes suitable for the amplification and detection of internal control gene amplification product, and the negative control(s), positive control(s), test sample(s), or standard samples (typically serially-diluted nucleic acid extract from a known bacterial species). For example, qRT-PCR of *E. coli* was performed using primer pair of SEQ ID NO: 7 and 8 and probe of SEQ ID NO: 19 and primer pair of SEQ ID NO: 29 and 30 and probe of SEQ ID NO: 31. In another example, qRT-PCR of *Enterococcus spp.* was performed using primer pairs of SEQ ID NO: 15 and 16 and SEQ ID NO: 17 and 18 and probes of SEQ ID NO: 23 and 24 respectively. The total reaction volume was typically adjusted from 10 to 20 µl using sterile water. The concentration of each of the reaction components can be determined empirically, but typically will comprise from about 1.5 to about 6 mM manganese acetate, about 0.2 to 0.6 mM of each dNTP, and suitable buffer 1X. Methods for optimizing and performing RTqPCR analyses are well known in the art and additional guidance is provided elsewhere herein. Further, premixed reagents can be conveniently obtained from a variety of commercial sources as described elsewhere herein. The cycling conditions are as followed: 1 cycle at 90° C for 15 seconds, 1 cycle at 59° C for 20 minutes, 1 cycle at 92° C for 30 seconds, followed by 40 cycles of 92° C for 5 sec and 56° C for 20 sec. The fluorescence was measured during the 56°C cycle.

### 6. RT Digital PCR

Digital PCR (dPCR) is a method for performing digital PCR that is based on water-oil emulsion droplet technology.

A reaction mixture is prepared comprising dNTPs, manganese acetate (or suitable cation), reaction buffer, enzyme with both DNA polymerase and reverse transcriptase activity like Tth, primers and probes as previously indicated for qRT-PCR, and the negative control(s), positive control(s), test sample(s), or standard samples (typically serially-diluted nucleic acid extract from a known bacterial species). The total reaction volume was typically adjusted from 10 to 25 µl using sterile water. The concentration of each of the reaction components can be determined empirically, but typically will comprise from about 1.5 to about 6 mM manganese acetate, about 0.2 to 0.6 mM of each dNTP, and suitable buffer 1X.

The reaction mixture is then fractionated into 20 000 droplets, into the commercial droplet generator, and RT-PCR amplification of the template molecules occurs in each individual droplet.

Methods for optimizing and performing qRT-PCR analyses are well known in the art and additional guidance is provided elsewhere herein. Further, premixed reagents can be conveniently obtained from a variety of commercial sources as described elsewhere herein. The cycling conditions are as followed: 1 cycle at 90° C for 15 seconds, 1 cycle at 59° C for 20 minutes (RT), 1 cycle at 92° C for 30 seconds followed by 40 cycles of 92° C for 5 sec and 56° C for 20 sec. The fluorescence is measured during the 56°C cycle and read in a specific commercial droplet reader.

### 7. RNA data analysis

After qRT-PCR, the results may be analysed using the delta Ct method (see Haugland et al., 2005, supra) or by directly extrapolating from a standard curve generated according to known methods using the results from the serially-diluted standard nucleic acid samples according to the following table :

**Table 6**

| **Well** | **Fluor** | **Target** | **Content** | **Sample** | **Cq** | **SQ** |
|---|---|---|---|---|---|---|
| A01 | FAM | E coli | Std-1 | | 22,09 | 250000 |
| A02 | FAM | E coli | Std-2 | | 25,50 | 25000 |
| A03 | FAM | E coli | Std-3 | | 30,77 | 2500 |
| A05 | FAM | E coli | Neg Ctrl | | 38,09 | 43 |
| A06 | FAM | E coli | Unkn | sample 1 | 29,57 | 4005 |
| A07 | FAM | E coli | Unkn | sample 2 | 31,90 | 1165 |
| A08 | FAM | E coli | Unkn | sample 3 | 31,38 | 1534 |
| A09 | FAM | E coli | Unkn | sample 4 | 31,18 | 1705 |
| A10 | FAM | E coli | Unkn | sample 5 | 31,19 | 1697 |
| A11 | FAM | E coli | Unkn | sample 6 | 31,20 | 1686 |
| A12 | FAM | E coli | Unkn | sample 7 | 31,02 | 1851 |
| B01 | FAM | E coli | Unkn | sample 8 | 31,00 | 1870 |
| B02 | FAM | E coli | Unkn | sample 9 | 31,50 | 1438 |
| B03 | FAM | E coli | Unkn | sample 10 | 28,32 | 7767 |
| B04 | FAM | E coli | Unkn | sample 11 | 31,46 | 1471 |
| B05 | FAM | E coli | Unkn | sample 12 | 35,58 | 165 |
| B06 | FAM | E coli | Unkn | sample 13 | 37,92 | 48 |
| B07 | FAM | E coli | Unkn | sample 14 | 30,39 | 2588 |
| B08 | FAM | E coli | Unkn | sample 15 | 39,81 | 17 |
| B09 | FAM | E coli | Unkn | sample 16 | | 0 |
| B10 | FAM | E coli | Unkn | sample 17 | 30,25 | 2795 |

After Digital RT-PCR, the results may be analysed using the absolute count of target copies per imput sample without the need for running standard curves.

**Table 7**

| **Chamber Name** | **ChamberID** | **Sample Name** | **TotalNumber Of Droplets** | **Blue Channel Concentration** | **Blue Channel NumberOf Positive Droplets** |
|---|---|---|---|---|---|
| A1 | 04222910-A1 | Ec10 000 CFU | 8645 | 24,7 | 94 |
| B1 | 04222910-B1 | Ec1000 CFU | 8997 | 7,05 | 28 |
| C1 | 04222910-C1 | Ec100 CFU | 9196 | 1,48 | 6 |
| D1 | 042229-0-D1 | T Neg | 10885 | 0 | 0 |

### 8. Results

The performance of the traditional IDEXX defined-substrate methods for *E. coli* and enterococci are compared to the method of the present invention. Colilert™ and Enterolert™ test kits were used according to guidelines from the manufacturer. Briefly, the reagent is added to the water sample in a sterile, transparent, non-fluorescent vessel and then poured into the Quanti-trays. Quanti-trays were sealed and then incubated for 24 hours at 35°C (*E. coli*) or 41°C (enterococci). Trays were then compared to comparators, and positive wells were counted and transformed to determine most probable numbers (MPNs) using the provided MPN chart.

Colilert™ Reagent is used for the simultaneous detection and confirmation of total coliforms and *E.coli* in water. It is based on IDEXX's patented Defined Substrate Technology® (DST™) This product utilizes nutrient indicators that produce colour(yellow wells for total coliforms) and/or fluorescence (fluorescent wells for *E. coli*) when metabolized by total coliforms and *E. coli.* When the reagent is added to the sample and incubated, it can detect these bacteria at 1CFU/100 ml within 24 hours.

Enterolert™ detects enterococci and is based on IDEXX's patented Defined Substrate Technology® (DST®). When enterococci utilize their β-glucosidase enzyme to metabolize Enterolert's nutrient-indicator, 4-methyl-umbelliferyl β-D-glucoside, the sample fluoresces. Enterolert detects enterococci at 1 CFU per 100 ml sample within 24 hours.

Q-RT-PCR was performed according to the method of the invention for *E.coli* and *Enterococcus spp.*

Comparative results obtained with Colilert IDEXX method and the method of the invention are illustrated hereafter.

**Table 8**

| **Sample number** | **Colilert Idexx** | **Q RT-PCR** |
|---|---|---|
| | **CFU of *E.coli*** | **CFU of *E.coli*** |
| | **24 hours analysis time** | **4 hours analysis time** |
| 2798 | 1607 | 2376 |
| 2799 | 609 | 759 |
| 2804-1 | 1354 | 1964 |
| 2804-2 | 1354 | 2358 |
| 2825 | 1046 | 732 |
| 2994 | 1039 | 623 |
| 3512 | 443 | 546 |
| 3551 | 9 | 0 |
| 3554 | 9 | 0 |
| 3595 | 945 | 1314 |
| 1117 | 156 | 111 |
| 1118 | 109 | 91 |
| 1214 | 439 | 269 |
| 2623 | 907 | 619 |
| 2624 | 19863 | 10789 |
| 1028 | 867 | 623 |
| 1029 | 565 | 898 |
| 1030 | 121 | 197 |
| 1031 | 359 | 550 |
| 1465 | 450 | 214 |
| 1673 | 1081 | 666 |
| 1695 | 309 | 787 |
| 2102 | 1284 | 1063 |
| 3393 | 813 | 534 |
| 1602 | 31 | 0 |
| 1094 | 487 | 666 |
| 1099 | 63 | 116 |
| 1122 | 798 | 1212 |
| 1126 | 657 | 579 |
| 1147 | 987 | 614 |
| 2407 | 108 | 105 |
| 3316 | 459 | 628 |
| 2343 | 0 | 40 |
| 2852 | 364 | 199 |
| 2853 | 253 | 62 |

There are no discrepancies between the reference Colilert IDEXX and the method of the invention. The method of the invention allows a good discrimination between samples containing from 0 to 2.10⁴ CFU of *E. coli*/100ml, thus enabling discrimination between "poor" and "good" quality water. However, results can be obtained in about 4 hours with the method of the invention.

Comparative results obtained with Enterolert IDEXX method and the method of the invention are illustrated hereafter.

**Table 9**

| **Sample number** | **Enterolert Idexx** | **RtqPCR** |
|---|---|---|
| | **CFU of *Enterococcus spp.*** | **CFU of *Enterococcus spp.*** |
| | **24 hours analysis time** | **4 hours analysis time** |
| 2798 | 1334 | 790 |
| 2804-1 | 1187 | 881 |
| 2805 | 345 | 335 |
| 2524 | 495 | 447 |
| 2825 | 512 | 709 |
| 2994 | 677 | 447 |
| 3312 | 1779 | 1021 |
| 3313 | 269 | 381 |
| 3539 | 9 | 42 |
| 3551 | 20 | 22 |
| 3554 | 9 | 18 |
| 3555 | 609 | 367 |
| 3558 | 30 | 16 |
| 3575 | 588 | 848 |
| 3591 | 341 | 552 |
| 3594 | 9 | 72 |
| 3595 | 489 | 201 |
| 1112 | 1497 | 1131 |
| 1113 | 1153 | 904 |
| 1114 | 620 | 468 |
| 1116 | 106 | 141 |
| 1117 | 41 | 141 |
| 1118 | 41 | 146 |
| 1119 | 20 | 146 |
| 1214 | 313 | 511 |
| 1215 | 520 | 811 |
| 1216 | 98 | 151 |
| 2803 | 158 | 277 |
| 2102 | 733 | 556 |
| 1099 | 97 | 54 |
| 3316 | 275 | 165 |

There are no discrepancies between the reference Enterolert IDEXX and the method of the invention. The method of the invention allows a good discrimination between samples containing from 10 to 2.10³ CFU of *Enterococcus spp.*/100ml, thus enabling discrimination between "poor" and "good" quality water. However, results can be obtained in about 4 hours with the method of the invention.

It is worth noting that 200-500 CFU/100 ml are generally considered as an area of values where the discriminatory performance is 'insufficient' (also called "grey zone"), in the sense that a value in the grey zone does not allow the exact number of FIB to be scored. This grey zone is closely related to the microbiological enumerations of the "poor quality water" as defined by Directive 2006/7/EC.

As shown in tables 8 and 9, the method of the invention permits the counting of the FIB even in the grey zone as previously defined, thus illustrating the reliability of the assays.

## Claims

1. A method for detecting and/or quantifying living and viable fecal indicator bacteria in a sample, comprising the steps of:
a) Providing a water sample,
b) Isolating ribonucleic acid (RNA) from said sample,
c) Performing a reverse transcription polymerase chain reaction (RT-PCR) amplification reaction with a mix comprising the isolated RNA, suitable PCR primer pairs capable of amplifying at least one nucleic acid target sequence selected from the group consisting of SEQ ID NO: 1, SEQ ID NO:2, SEQ ID NO: 3, SEQ ID NO:4, SEQ ID NO:5 and SEQ ID NO:6,
d) Detecting and/or quantifying the RT-PCR amplifications products, wherein amplification of SEQ ID NO: 1, SEQ ID NO: 2, SEQ ID NO: 3 or SEQ ID NO:4 is indicative of the presence of *E. coli* in said sample and wherein amplification of SEQ ID NO:5 or SEQ ID NO:6 is indicative of the presence of *Enterococcus spp.* in said sample.

2. The method of claim 1, wherein said suitable primer pairs consist of any combination of a forward primer selected from the group consisting of SEQ ID NO: 7, SEQ ID NO: 9, SEQ ID NO: 11, SEQ ID NO: 13 and their complementary sequences, a reverse primer selected from the group consisting of SEQ ID NO: 8, SEQ ID NO: 10, SEQ ID NO: 12, SEQ ID NO: 14 and their complementary sequences, and a forward and a reverse primer comprising at least a contiguous sequence of approximately at least about 15 nucleotides, preferably at least about 17-18 nucleotides, and more preferably at least about 19-22 nucleotides of SEQ ID NO: 7 to 14 and their complementarity sequences.

3. The method of claim 1 or 2, wherein said suitable primer pair consists of any combination of a forward primer selected from the group consisting of SEQ ID NO: 15, SEQ ID NO: 17 and their complementary sequences, a reverse primer selected from the group consisting of SEQ ID NO: 16, SEQ ID NO: 18 and their complementary sequences, and a forward and a reverse primer comprising at least a contiguous sequence of approximately at least about 13 nucleotides, preferably at least about 20-21 nucleotides of SEQ ID NO: 15 to 18 and their complementary sequence.

4. The method of claims 2 or 3, wherein the mix of step c) further comprises at least one probe selected from the group consisting of SEQ ID NO: 19, SEQ ID NO: 20, SEQ ID NO: 21, SEQ ID NO: 22, SEQ ID NO: 23 and SEQ ID NO: 24.

5. The method of any of claims 1 to 4, further comprising the step of identifying and/or quantifying *Staphylococcus aureus,* wherein the mix of step c) also comprises at least one suitable PCR primer pair capable of amplifying a nucleic acid target sequence of SEQ ID NO: 28 and wherein amplification of SEQ ID NO: 28 is indicative of the presence of *Staphylococcus aureus* in said sample.

6. The method of any of claims 1 to 5, further comprising a step of filtrating and collecting bacteria prior to step b).

7. The method of any of claims 1 to 6, further comprising a bacterial DNA elimination, so as to obtain pure RNA, prior to step c).

8. The method of any of claims 1 to 6, wherein the RT-PCR amplification reaction can be a real time reverse-transcription-polymerase chain reaction (RT-PCR) or a digital RT-PCR.

9. The method of any of claims 1 to 8, wherein the amplification product is analyzed by using a standard curve presenting a relationship between an amount of the living and viable fecal indicators bacteria and the amplification product, prepared by using standard samples of fecal indicator bacteria.

10. The method of any of claims 1 to 9, **characterized in that** RT-PCR is carried out on a sample containing a population of *E. coli* from 10 to 10⁷ CFU/100 ml.

11. The method of any of claims 1 to 10, wherein the water sample is selected from the group consisting of ground water, spring water, surface water, sea water, waste water, seepage water, drinking water, groundwater bodies, springs, water distribution system, rivers, lakes, marine environments, sewage and waste water treatment systems.

12. A kit for the detection and/or quantification of FIB in a water sample comprising means for the detection of at least one target sequence of SEQ ID NO: 1, SEQ ID NO: 2, SEQ ID NO: 3, SEQ ID NO: 4, SEQ ID NO: 5 or SEQ ID NO:6 and instructions how to use the kit.

13. The kit of claim 12, wherein means for the detection of at least one target sequence of SEQ ID NO: 1, SEQ ID NO: 2, SEQ ID NO: 3, SEQ ID NO: 4, SEQ ID NO: 5 or SEQ ID NO: 6 comprise at least one primer pair selected from the group consisting of SEQ ID NO: 7 and 8, SEQ ID NO: 9 and 10, SEQ ID NO: 11 and 12, SEQ ID NO: 13 and 14, SEQ ID NO: 15 and 16 and SEQ ID NO: 17 and 18.

14. The kit of claim 13, wherein means for the detection of at least one target sequence of SEQ ID NO: 1, SEQ ID NO: 2, SEQ ID NO: 3, SEQ ID NO: 4, SEQ ID NO: 5 or SEQ ID NO: 6 further comprise at least one probe selected from the group consisting of SEQ ID NO: 19, SEQ ID NO: 20, SEQ ID NO: 21, SEQ ID NO: 22, SEQ ID NO: 23 and SEQ ID NO: 24.

15. An amplicon obtainable by amplification from a fecal indicator bacteria-containing water sample with a forward primer of SEQ ID NO: 7 and a reverse primer of SEQ ID NO: 8, wherein said amplicon is bound to a probe comprising a fluorophore-quencher pair, wherein said probe is of SEQ ID NO: 19.

16. An amplicon obtainable by amplification from a fecal indicator bacteria-containing water sample with a forward primer of SEQ ID NO: 15 and a reverse primer of SEQ ID NO: 16, wherein said amplicon is bound to a probe comprising a fluorophore-quencher pair, wherein said probe is of SEQ ID NO: 23.

17. A primer set for amplifying a fecal indicator bacteria polynucleotide comprising one or more primer pair selected from the group consisting of SEQ ID NO: 7 and 8, SEQ ID NO: 9 and 10, SEQ ID NO: 11 and 12, SEQ ID NO: 13 and 14, SEQ ID NO: 15 and 16, and SEQ ID NO: 17 and 18.
